(19) Europäisches Patentamt / European Patent Office / Office européen des brevets

(11) **EP 4 620 497 A1**

(12) **EUROPEAN PATENT APPLICATION**

(43) Date of publication:
24.09.2025 Bulletin 2025/39

(21) Application number: 25162698.2

(22) Date of filing: 10.03.2025

(51) International Patent Classification (IPC):
*A61M 1/36* (2006.01)   *A61M 60/109* (2021.01)
*A61M 60/232* (2021.01)   *A61M 60/38* (2021.01)
*A61M 60/508* (2021.01)   *A61M 60/585* (2021.01)
*A61M 60/825* (2021.01)   *F04D 15/00* (2006.01)
*F04D 15/02* (2006.01)   *F04D 29/046* (2006.01)
*G16H 40/20* (2018.01)

(52) Cooperative Patent Classification (CPC):
**A61M 60/109; A61M 1/3666; A61M 60/232;
A61M 60/38; A61M 60/508; A61M 60/585;
A61M 60/825; F04D 15/0088; F04D 15/0272;
F04D 29/0467; G16H 40/20;** A61M 2205/18;
A61M 2205/50; A61M 2205/502; A61M 2205/52

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 11.03.2024 CN 202410271790

(71) Applicant: **ChinaBridge (Shenzen) Medical
Technology Co., Ltd.**
**Shenzhen, Guangdong 518102 (CN)**

(72) Inventor: **LI, Yijiang**
**Shenzhen Guangdong, 518102 (CN)**

(74) Representative: **Pfenning, Meinig & Partner mbB
Patent- und Rechtsanwälte
Joachimsthaler Straße 10-12
10719 Berlin (DE)**

(54) **METHOD AND DEVICE FOR PREDICTING BLOOD PUMP REMAINING AVAILABLE TIME OF CENTRIFUGAL PUMP, AND APPARATUS AND MEDIUM**

(57)    A method for predicting a blood pump remaining available time includes: obtaining a duration of a current monitoring cycle of the blood pump; obtaining a current cycle wear of a single ball bearing in the current monitoring cycle based on the current monitoring cycle; determining a current total wear of the single ball bearing based on the current cycle wear of the current monitoring cycle and historical cycle wears of historical monitoring cycles prior to the current monitoring cycle; determining a remaining available wear of the blood pump based on the current total wear and a preset total wear threshold; calculating an initial estimated available time of the blood pump based on the remaining available wear, the current cycle wear, and the duration of the current monitoring cycle; and performing a filtering process based on the calculated initial estimated available time to determine an ultimate estimated available time.

FIG. 5

EP 4 620 497 A1

**Description**

**Technical Field**

The present invention relates to the technical field of medical device monitoring, and in particular to a method and a device for predicting the blood pump remaining available time of a centrifugal pump, an apparatus and a medium.

**Background Art**

In clinical emergency treatment of critically ill patients with severe cardiopulmonary failure, extracorporeal membrane oxygenation (ECMO) is used to provide patients with continuous extracorporeal respiratory and circulatory support to gain more emergency time. The core parts of extracorporeal membrane oxygenation (ECMO) are artificial lungs (also known as membrane lungs or oxygenators) and artificial hearts (also known as blood pumps or power pumps). As the technologies related to artificial lungs and artificial hearts are becoming more and more sophisticated, ECMO can be maintained for a longer time, which also provides the prerequisite for ECMO to be used in the treatment of patients with cardiopulmonary failure.

The existing ECMO mainly achieves blood flow by driving the blood pump to rotate.

During ECMO treatment (e.g., surgery) in the clinic, due to differences in usage scenarios, the blood pump may experience various conditions such as pipe bending and bubbles after long-term operation. Usually during the use of the device, clinicians often use the constant speed mode, that is, the speed is set for a period of time, and then the device operates and monitors itself. However, in this mode, the different physical conditions of the patients can also cause the blood pump parameters such as pressure, flow, and speed to change. These changes may further cause the blood pump to wear to varying degrees. When the wear reaches a certain degree, it means that the blood pump has reached the limit of its service life. When the blood pump reaches the limit of its service life, a large deviation from normality will occur.

Most centrifugal blood pumps used in the ECMO field are composed of an external housing and an internal rotor. The rotation of the rotor drives the blood to flow in the blood pump. Due to the particularity of the ECMO centrifugal blood pump, the volume of the blood pump needs to be as small as possible to reduce the extracorporeal circuit volume. The speed of the centrifugal blood pump is often high. Due to the high speed, the force of supporting the liquid between the rotor and the housing and the load of the pump shaft are large, which can not only withstand large axial and radial load capacity, but also have high axial accuracy. Compared with general bearings, single ball bearings are more compact in size and more suitable for applications in limited spaces. As ball bearings roll rather than slide during operation, they are more suitable for high-speed rotation, and have higher axial accuracy and higher axial and radial load capacity. Therefore, a single ball bearing is usually used to connect the rotor and the housing. A prior art structure can be seen in CN111249551B. Existing common centrifugal pumps using single ball bearings include CN113446259A, CN112915293A, CN115300785A, WO2021051645A2, etc.

**In** a centrifugal blood pump equipped with a single ball bearing, the impeller rotates under the action of the fluid and often rubs against the single ball bearing. The wear on the single ball bearing is much greater than that on other components. Therefore, the service life of the blood pump is mainly affected by the wear of the single ball bearing. Therefore, the remaining wear of the single ball bearing can be regarded as the remaining service life of the blood pump.

The existing technology lacks a method for determining the service life of a centrifugal pump using a single ball bearing. The existing conventional method for predicting the remaining life of a blood pump is to compare the wear rate based on a mapping relationship between flow and speed or pressure, or to use the characteristic curve as an evaluation indicator for the failure life of a centrifugal pump, such as patents CN106089753B and CN110259702A. However, this method can only calculate the situation that matches the characteristic curve. When it does not match, it will be determined as a fault or failure. This approach is relatively limited and has a large error; in addition, it cannot take into account the situation where unexpected factors occur. For example, if the pipes connecting the two ends of the pump are bent or squeezed, the flow rate can change, causing the characteristic curve to not match and thus causing misjudgment.

When clinical medical staff use the equipment, there are usually fixed speed and fixed flow modes. The fixed speed mode is to set the speed, and the fixed flow mode is to set the flow. In the fixed speed mode, the flow and pressure may change. In the fixed flow mode, the speed and pressure may change. Due to the differences between ECMO systems of various manufacturers, generally speaking, when the ECMO system has a fixed flow mode, medical staff may prefer to use this mode, because the constant flow ensures that the flow does not change.

Due to differences in the processing technology of the blood pump itself, the use environment factors, the patient's physical condition, etc., the service life of the blood pump may vary. Since the blood pump is a high-value-added consumable, the same blood pump will be used as much as possible during extracorporeal circulation procedure. The operation time of the blood pump is often relatively long, and its actual service life varies depending on the environment. Clinical doctors and maintenance personnel of equipment manufacturers use experience to judge the use of blood pumps based on conditions such as flow rate and the sound of blood pump operation. This is not conducive to predicting the development of the disease and the status of the equipment for the hospital and maintenance manufacturers. Replacing the blood pump too early will cause waste of consumables, and replacing it too late will endanger the health of the patient and even affect life safety. Based on the above problems, we now add a monitoring function to predict the life of the blood pump to the equipment, which can more objectively display the remaining use time of the blood pump, so that medical staff can better control the rhythm of the entire procedure.

## Summary of the Invention

The embodiments of the present invention provide a method and a device for predicting a blood pump remaining available time of a centrifugal pump, a computer device and a storage medium, which aim to solve the problem that it is difficult to accurately determine the remaining available time of a blood pump in the existing technology, resulting in difficulty in determining the timing of blood pump replacement, and premature replacement of the blood pump will cause waste of consumables.

In a first aspect, the embodiments of the present invention provide a method for predicting a blood pump remaining available time of a centrifugal pump, which comprises a rotor and a housing, and the rotor and the housing are connected by a single ball bearing, the method comprising: obtaining a current monitoring cycle of the blood pump, and obtaining a cycle wear of the single ball bearing in the monitoring cycle based on the monitoring cycle; determining a current total wear of the single ball bearing based on the cycle wear of the monitoring cycle and historical cycle wears of historical monitoring cycles prior to the monitoring cycle; determining a remaining available wear of the blood pump based on the total wear and a preset total wear threshold; calculating an initial estimated available time of the blood pump based on the remaining available wear, the cycle wear and a duration of the monitoring cycle; and performing a filtering process based on the calculated initial estimated available time to determine an estimated available time of the blood pump.

Through the above technical solution, the remaining available time of the blood pump can be accurately predicted, so that a user can accurately grasp the timing of replacing the blood pump, which ensures safety on the one hand and avoids waste of consumables on the other.

A further technical solution thereof is as follows: the performing of the filtering process based on the calculated initial estimated available time to determine the estimated available time of the blood pump comprises: obtaining a historical estimated available time corresponding to a preceding historical monitoring cycle of the monitoring cycle; determining whether a difference between the historical estimated available time and the initial estimated available time is less than a preset first time threshold; and using the initial estimated available time as the estimated available time of the blood pump if the difference between the historical estimated available time and the estimated available time is smaller than the preset first time threshold.

Through the above technical solution, abnormal prediction situations can be accurately filtered out, so that the calculated estimated available time is accurate and reliable, thus the user can accurately understand the wear of the blood pump.

A further technical solution thereof is as follows: the obtaining of the cycle wear of the single ball bearing in a preset monitoring cycle comprises: obtaining a target speed value of the blood pump and a target pressure difference between an output port and an input port of the blood pump during the monitoring cycle; and determining the cycle wear based on the target speed value, the target pressure difference and the duration of the monitoring cycle.

The above technical solution can accurately determine the cycle wear within the monitoring cycle based on the target speed value of the blood pump within the monitoring cycle, the target pressure difference between the output port and the input port of the blood pump, and the duration of the monitoring cycle, so that the user can accurately understand the wear condition of the blood pump.

A further technical solution thereof is as follows: the obtaining of the target speed value of the blood pump and the target pressure difference between the output port and the input port of the blood pump during the monitoring cycle comprises: obtaining a weighted arithmetic mean or harmonic mean of speed values of the blood pump during the monitoring cycle as the target speed value; and obtaining a weighted arithmetic mean or harmonic mean of pressure differences between the output port and the input port of the blood pump during the monitoring cycle as the target pressure difference.

The above technical solution represents the overall situation of the rotation speed and pressure difference during the monitoring cycle based on the weighted arithmetic mean or the harmonic mean, so that the wear of the blood pump during the monitoring cycle can be accurately predicted even when the rotation speed or the pressure difference is unstable.

A further technical solution thereof is as follows: the determining of the cycle wear based on the target speed value, the target pressure difference and the duration of the monitoring cycle comprises: obtaining a preset mapping relationship between pre-stored speed values, pressure difference value as well as durations and wears; and determining the cycle wear corresponding to the target speed value, the target pressure difference value and the duration of the monitoring cycle based on the preset mapping relationship.

The above technical solution can quickly and accurately determine the cycle wear by means of directly calling a preset mapping relationship that is previously set.

A further technical solution thereof is as follows: the determining of the total wear of the single ball bearing based on the cycle wear of the monitoring cycle and the historical cycle wears of the historical monitoring cycles prior to the monitoring cycle comprises: obtaining the total wear by accumulating and summing the cycle wear and all the historical cycle wears.

The cycle wear and all the historical cycle wears are accumulated and summed to obtain the total wear.

A further technical solution thereof is as follows: the determining of the initial estimated available time of the blood pump based on the remaining available wear, the cycle wear and the duration of the monitoring cycle comprises: calculating a ratio of the remaining available wear to the cycle wear; and calculating a product of the ratio and the duration of the monitoring cycle to obtain the initial estimated available time of the blood pump.

The above technical solution assumes that the cycle wear of each subsequent monitoring cycle is the same, so by calculating the ratio of the remaining available wear to the cycle wear; and further calculating the product of the ratio and the duration of the monitoring cycle, the initial estimated available time of the blood pump can be accurately obtained.

A further technical solution thereof is as follows: the method further comprises: determining whether the estimated available time is less than a preset second time threshold; and issuing an alarm message if the estimated available time is less than the preset second time threshold.

The above technical solution can timely remind the staff before the blood pump is damaged by setting the second time threshold, thereby providing the staff with sufficient preparation time for replacing the blood pump.

In a second aspect, the embodiments of the present invention further provide a device for predicting a blood pump remaining available time of a centrifugal pump, the device comprises units for executing the method described above.

In a third aspect, the embodiments of the present invention further provide a computer device, the computer device comprises a memory and a processor, the memory stores a computer program, and the processor implements the method described above when executing the computer program.

In a fourth aspect, the embodiments of the present invention further provide a computer-readable storage medium, the storage medium stores a computer program, and when the computer program is executed by a processor, the method described above is implemented.

The embodiments of the present invention provide a method and a device for predicting a blood pump remaining available time of a centrifugal pump, a computer device and a storage medium. The method comprises: obtaining a current monitoring cycle of the blood pump, and obtaining a cycle wear of the single ball bearing in the monitoring cycle based on the monitoring cycle; determining a current total wear of the single ball bearing based on the cycle wear of the monitoring cycle and historical cycle wears of historical monitoring cycles prior to the monitoring cycle; determining a remaining available wear of the blood pump based on the total wear and a preset total wear threshold; calculating an initial estimated available time of the blood pump based on the remaining available wear, the cycle wear and a duration of the monitoring cycle; and performing a filtering process based on the calculated initial estimated available time to determine an estimated available time of the blood pump; and further filtering is performed based on the calculated initial estimated available time to determine the estimated available time of the blood pump, thereby accurately predicting the remaining available time of the blood pump, so that the user can accurately grasp the timing of replacing the blood pump, which ensures safety on the one hand and avoids waste of consumables on the other.

**Brief Description of the Drawings**

In order to more clearly illustrate the technical solutions of the embodiments of the present invention, the following will briefly introduce the drawings required for the description of the embodiments. Obviously, the drawings described below are some embodiments of the present invention. For a person of ordinary skill in the art, other drawings can be obtained based on these drawings without creative work.

FIG. 1 is a flow chart of a method for predicting a blood pump remaining available time of a centrifugal pump provided in an embodiment of the present invention;

FIG. 2 is a structural diagram of a blood pump of a centrifugal pump provided in an embodiment of the present invention;

FIG. 3 is another flow chart of a method for predicting a blood pump remaining available time of a centrifugal pump provided in another embodiment of the present invention;

FIG. 4 is a schematic block diagram of a device for predicting a blood pump remaining available time of a centrifugal pump provided in an embodiment of the present invention; and

FIG. 5 is a schematic block diagram of a computer device provided in an embodiment of the present invention.

**Description of the Embodiments**

The following will clearly and fully describe the technical solutions in the embodiments of the present invention in conjunction with the drawings in the embodiments of the present invention. Obviously, the described embodiments are part of the embodiments of the present invention, not all of them. Based on the embodiments of the present invention, all other embodiments obtained by a person of ordinary skill in the art without creative work are within the scope of protection of the present invention.

It should be understood that when used in this specification and the appended claims, the terms "include" and "comprise" indicate the presence of the described features, wholes, steps, operations, elements and/or components, but do not exclude the presence or addition of one or more other features, wholes, steps, operations, elements, components and/or their collections.

It should also be understood that the terms used in this description of the present invention are only for the purpose of describing specific embodiments and are not intended to limit the present invention. As used in the specification of the present invention and the appended claims, the singular forms "a," "an" and "the" are intended to include the plural forms unless the context clearly indicates otherwise.

It should be further understood that the term "and/or" used in the present description and the appended claims refers to any combination of one or more of the associated listed items and all possible combinations, and includes these combinations.

As used in the present description and the appended claims, the term "if" can be interpreted as "when ..." or "once" or "in response to determination" or "in response to detection" depending on the context. Similarly, the phrase "if it is determined" or "if [the described condition or event] is detected" can be interpreted as meaning "once it is determined" or "in response to determination" or "once [the described condition or event] is detected" or "in response to detection of [the described condition or event]" depending on the context.

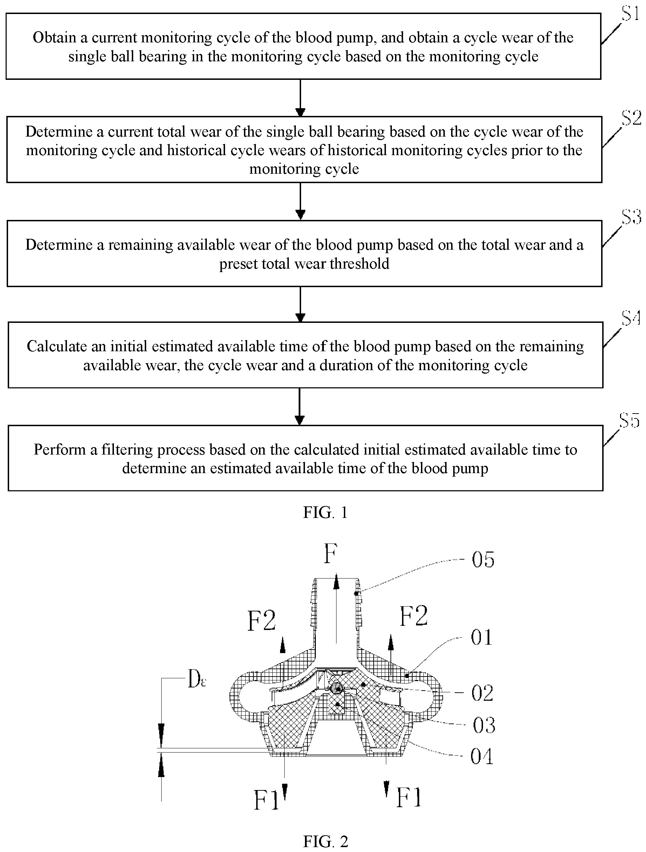

With reference to FIG. 1, an embodiment of the present invention provides a method for predicting a blood pump remaining available time of a centrifugal pump, which can be applied to ECMO equipment. The blood pump of a centrifugal pump includes a rotor and a housing, and the rotor and the housing are connected by a single ball bearing. The method includes the following steps S1 to S5.

S1. Obtain a current monitoring cycle of the blood pump, and obtain a cycle wear of the single ball bearing in the monitoring cycle based on the monitoring cycle.

In a specific implementation, the current monitoring cycle of the blood pump is first determined, for example, the monitoring cycle is set to 10 minutes, that is, every 10 minutes is a monitoring cycle. In the embodiment of the present invention, the monitoring cycle can be preset by a user, or adaptively adjusted according to the remaining computing power of the monitoring device. That is, the mapping relationship between the remaining computing power and the monitoring cycle is set, and the monitoring cycle can be automatically determined according to the remaining computing power. Generally speaking, the more the remaining computing power, the shorter the monitoring cycle can be set, thereby achieving more accurate monitoring.

Alternatively, the monitoring cycle can be adaptively adjusted according to the speed value of the blood pump and the pressure difference between the output port and the input port of the blood pump. That is, the mapping relationship between the speed value, the pressure difference and the monitoring cycle is set, and the monitoring cycle can be automatically determined according to the speed value and the pressure difference. Generally speaking, the larger the speed value and the pressure difference, the shorter the monitoring cycle can be set, thereby achieving more accurate monitoring.

After the monitoring cycle ends, the cycle wear of the monitoring cycle is obtained, and the cycle wear herein refers to the wear amount of the single ball bearing of the blood pump in a monitoring cycle.

In one embodiment, the above step of "obtaining the cycle wear of the single ball bearing in the preset monitoring cycle" specifically includes the following steps:

S11. Obtain a target speed value of the blood pump and a target pressure difference value between an output port and an input port of the blood pump within the monitoring cycle.

In a specific implementation, the wear amount of a single ball bearing is related to the rotation speed of the blood pump and the pressure difference between the output port and the input port of the blood pump. In an ECMO device, the output port of the blood pump refers to the blood outlet port. The input port of the blood pump refers to the blood inlet port. The pressures at the output port and the input port of the blood pump are measured by a pressure sensor. The rotation speed of the blood pump is usually set by the user.

When the ECMO device is running stably, the rotation speed of the blood pump and the pressures at the output port and the input port of the blood pump are usually constant or change very little. Therefore, in an embodiment of the present invention, the target rotation speed value of the blood pump and the pressure values at the output port and the input port of the blood pump during the monitoring cycle are usually directly read, and the target pressure difference between the output port and the input port of the blood pump is further calculated.

Under some working conditions (e.g., regulating flow), the speed of the blood pump and the pressure of the output and input ports of the blood pump will change. Accordingly, in order to accurately obtain the cycle wear of the single ball bearing of the blood pump, in one embodiment, the above step of "obtaining the target speed value of the blood pump and the target pressure difference between the output and input ports of the blood pump during the monitoring cycle" specifically includes the following steps:

S111. Obtain a weighted arithmetic mean or harmonic mean of speed values of the blood pump during the monitoring cycle as the target speed value.

In a specific implementation, the blood pump speed value is collected once at every preset sampling cycle within the monitoring cycle, and then the weighted arithmetic mean or harmonic mean of the collected speed values is calculated as the target speed value.

S112. Obtain a weighted arithmetic mean or harmonic mean of pressure differences between the output port and the input port of the blood pump during the monitoring cycle as the target pressure difference.

In a specific implementation, the pressure difference between the output port and the input port is collected once at every preset sampling cycle within the monitoring cycle, and then the weighted arithmetic mean or harmonic mean of the collected pressure difference is calculated as the target pressure difference.

S12. Determine the cycle wear based on the target speed value, the target pressure difference and the duration of the monitoring cycle

In a specific implementation, the wear amount of the blood pump is related to the rotation speed value of the blood pump, the pressure difference between the output port and the input port of the blood pump, and the operation time/duration of the blood pump. Therefore, in an embodiment of the present invention, the cycle wear of the monitoring cycle is determined by the target speed value, the target pressure difference value, and the duration of the monitoring cycle of the blood pump in the monitoring cycle.

For example, in one embodiment, the above step of "determining the cycle wear based on the target speed value and the target pressure difference value" specifically includes the following steps:

S121. Obtain a preset mapping relationship between pre-stored speed values, pressure difference value as well as durations and wear amounts.

In a specific implementation, the preset mapping relationship between the speed value, pressure difference value, duration and wear of the blood pump is obtained in advance. The preset mapping relationship can be obtained by function fitting, which is not specifically limited in the present invention.

S122. Determine the cycle wear corresponding to the target speed value, the target pressure difference value and the duration of the monitoring cycle based on the preset mapping relationship.

In a specific implementation, the target speed value, the target pressure difference value and the duration of the monitoring cycle are brought into the preset mapping relationship to calculate the cycle wear.

For example, in one embodiment, the preset mapping relationship is the following formula (1):

$$y = (k_1 \frac{n * (F_1 - \frac{\pi}{4}(D_e^2 - d_h^2) \triangle P) * sin2a}{40R(1 - cos^3 a_1)} t$$
$$+ k_2 \frac{n(F_1 - \frac{\pi}{4}(D_e^2 - d_h^2) \triangle P) sin 2a}{40R(1 - COS^3 a_1)} t) \cos a \qquad \text{formula (1)}$$

In the above formula, y is the cycle wear, $k_1$ is the wear coefficient of the ball of the single ball bearing, $k_2$ is the wear coefficient of the bearing, R is the radius of the ball (i.e. the ball of the single ball bearing), $F_1$ is the magnetic attraction of the blood pump, and $F_1$ is a fixed value, a1 is the angle between the radius corresponding to the midpoint of the ball socket (ball bearing) in the ball and the radius corresponding to the starting point of the ball socket, a is the angle between the radius of any point on the spherical surface on the contact surface in the ball and the radius corresponding to the midpoint of the ball socket (generally speaking, the size of a can be controlled by the structure, such as opening a countersink at the bottom of the spherical surface or milling a plane on the end face of the ball head, on the one hand, this can make the two not contact at this location, on the other hand, lubricating grease can be stored to reduce the friction coefficient of the contact surface), $D_e$ is the gap diameter of the impeller ring, $d_h$ is the diameter of the impeller hub, $\Delta P$ is the target pressure difference, n is the target speed value, and t is the duration of the monitoring cycle; among them, except $\Delta P$, n and t, other factors are fixed values. Therefore, by substituting $\Delta P$, n, and t, the cycle wear y can be obtained.

In order to better illustrate the technical solution of the present invention, the structure of the blood pump of the centrifugal pump of the embodiments of the present invention is shown in FIG. 2, in which 01 refers to the outer housing of the blood pump; 02 refers to the impeller, i.e., the rotor; 03 refers to the single ball bearing; 04 refers to the support of the single ball bearing; 05 refers to the blood inlet. F1 refers to the magnetic attraction force exerted on the impeller of the blood pump, and F2 refers to the pressure exerted on the impeller of the blood pump by the blood. F refers to the support force exerted on the impeller of the blood pump by the support; $D_e$ is the gap diameter of the impeller ring.

Now, in conjunction with FIG. 2, the derivation process of the above formula (1) is described as follows:

Based on the disclosures in "Analysis of Wear Characteristics of Spherical Friction Pairs" published by Mining Machinery Press (the article only proposes the formula algorithm in static mode, and the various variable parameters in this case are dynamic values):

$$\delta = \delta_1 + \delta_2 = h/cos a \qquad \text{formula (2)}$$

$$\delta_1 = k_1 \frac{3F}{2\pi R^2 (1 - cos^3 a_1)} \cos a \frac{n\pi}{30} R \sin at$$
$$= k_1 \frac{nF sin 2a}{40R(1 - cos^3 a_1)} t \qquad \text{formula (3)}$$

$$\delta_2 = k_2 \frac{nF \sin 2a}{40R(1 - COS^3 a_1)} t \qquad \text{formula (4)}$$

$$y = h = (\cos a)(\delta_1 + \delta_2) \qquad \text{formula (5)}$$

In the above formulae, $\delta$ is the total wear, $\delta_1$ and $\delta_2$ are the normal direction wear of the ball (i.e. the ball of a single ball bearing) and the socket (ball bearing) at a given point, respectively, y and h are the cycle wear, $k_1$ is the wear coefficient of the ball of a single ball bearing, $k_2$ is the wear coefficient of the bearing, R is the radius of the ball (i.e. the ball of a single ball bearing), a1 is the angle between the radius corresponding to the midpoint of the socket in the ball and the radius corresponding to the starting point of the socket, a is the angle between the radius of any point on the spherical surface of the ball and the radius corresponding to the midpoint of the socket, and when a=a1, it means the most serious wear, n is the target speed value, t is the duration of the monitoring cycle, and F is the axial force.

The axial force F exerted on the blood pump is mainly composed of the magnetic attraction force and the blood force, as shown in the following formula (6):

$$F = F_1 - F_2 \qquad \text{formula (6)}$$

In the above formula, $F_1$ is the magnetic attraction, $F_2$ is the force of blood, and the magnetic attraction of the pump head is fixed and is a known parameter. According to the principle of "Axial force, radial force and its balance" in Chapter 8 of "Vane Pump Principle and Hydraulic Design," the following can be obtained:

$$F_2 = \frac{\pi}{4}(D_e^2 - d_h^2)\Delta P \qquad \text{formula (7)}$$

In the above formula, $D_e$ is the gap diameter of the impeller ring, $d_h$ is the diameter of the impeller hub, and $\Delta P$ is the target pressure difference.

Based on the above formulae (2) to (7), the above formula (1) can be derived.

S2. Determine a current total wear of the single ball bearing based on the cycle wear of the monitoring cycle and historical cycle wears of historical monitoring cycles prior to the monitoring cycle.

In a specific implementation, the historical monitoring cycle refers to the monitoring cycle(s) before the current monitoring cycle. It can be understood that after a monitoring cycle ends, this monitoring cycle becomes a historical monitoring cycle, and at the same time, the cycle wear amount of this monitoring cycle becomes a historical cycle wear. In the embodiments of the present invention, the historical cycle wear of each historical monitoring cycle is stored.

Therefore, after determining the cycle wear of the current monitoring cycle, the cycle wear and all the historical cycle wears can be accumulated and summed to obtain the total wear.

S3. Determine a remaining available wear of the blood pump based on the total wear and a preset total wear threshold.

In a specific implementation, the total wear threshold is usually measured by the manufacturer. When the wear of a single ball bearing of the blood pump reaches the total wear threshold, it indicates that the blood pump is damaged and needs to be replaced.

In the embodiments of the present invention, the difference between the preset total wear threshold and the total wear is calculated to obtain the remaining available wear of the blood pump.

S4. Calculate an initial estimated available time of the blood pump based on the remaining available wear, the cycle wear and a duration of the monitoring cycle.

In a specific implementation, the initial estimated available time of the blood pump can be calculated by the remaining available wear, the cycle wear and the duration of the monitoring cycle. For example, the reason for using cycle monitoring here is that under normal circumstances, when the ECMO device is running stably, the speed of the blood pump and the pressure of the output port and the input port of the blood pump are usually constant or change very little, and the cycle wear of the single ball bearing of the blood pump can be considered to be constant. The cycle can be set very short, or the monitoring cycle can be automatically determined based on the remaining computing power.

Therefore, in one embodiment, the above step of "determining the initial estimated available time of the blood pump based on the remaining available wear, the cycle wear and the duration of the monitoring cycle" specifically includes the following steps:

S41. Calculate a ratio of the remaining available wear to the cycle wear.

In a specific implementation, the remaining available wear is divided by the cycle wear to obtain a ratio of the remaining available wear to the cycle wear.

S42. Calculate a product of the ratio and the duration of the monitoring cycle to obtain the initial estimated available time of the blood pump.

In a specific implementation, the product of the ratio and the duration of the monitoring cycle is calculated to obtain the initial estimated available time of the blood pump. For example, in one embodiment, the monitoring cycle is 10 minutes, the ratio is 100, and the initial estimated available time of the blood pump is 1000 minutes.

S5. Perform a filtering process based on the calculated initial estimated available time to determine an estimated available time of the blood pump.

In a specific implementation, after the initial estimated available time is calculated, filtering processing is further performed to determine the estimated available time of the blood pump.

For example, in one embodiment, the above step of "performing filtering processing based on the calculated initial estimated available time to determine the estimated available time of the blood pump" specifically includes the following steps:

S51. Obtain a historical estimated available time corresponding to a preceding historical monitoring cycle of the monitoring cycle.

In a specific implementation, the historical estimated available time corresponding to the previous/preceding historical monitoring cycle of the pre-stored monitoring cycle is obtained.

S52. Determine whether a difference between the historical estimated available time and the initial estimated available time is less than a preset first time threshold.

In a specific implementation, it is determined whether the difference between the historical estimated available time and the estimated available time is less than the preset first time threshold. The first time threshold can be set by a person skilled in the art, and the present invention does not specifically limit it. For example, the first time threshold can be set to 10 times the monitoring cycle, where the monitoring cycle is 10 minutes, and then the time threshold is 100 minutes. The purpose of setting the first time threshold is to filter abnormal situations.

S53. Use the initial estimated available time as the estimated available time of the blood pump if the difference between the historical estimated available time and the initial estimated available time is smaller than the preset first time threshold.

In a specific implementation, if the difference between the historical estimated available time and the estimated available time is less than the preset first time threshold, it means that the estimated available time is reliable. Therefore, the initial estimated available time is used as the estimated available time of the blood pump. At the same time, the remaining available time of the blood pump is updated to the estimated available time, and the updated remaining available time is displayed to the user.

Furthermore, if the difference between the historical estimated available time and the estimated available time is not less than the preset first time threshold, an abnormal prompt message can be issued. For example, "the data changes greatly and cannot be calculated" can be displayed to remind the user to pay attention to the abnormality.

The technical solution proposed in the embodiments of the present invention is as follows: obtaining a current monitoring cycle of the blood pump, and obtaining a cycle wear of the single ball bearing in the monitoring cycle based on the monitoring cycle; determining a current total wear of the single ball bearing based on the cycle wear of the monitoring cycle and historical cycle wears of historical monitoring cycles prior to the monitoring cycle; determining a remaining available wear of the blood pump based on the total wear and a preset total wear threshold; calculating an initial estimated available time of the blood pump based on the remaining available wear, the cycle wear and a duration of the monitoring cycle; and performing a filtering process based on the calculated initial estimated available time to determine an estimated available time of the blood pump; and further filtering is performed based on the calculated initial estimated available time to determine the estimated available time of the blood pump, thereby accurately predicting the remaining available time of the blood pump, so that the user can accurately grasp the timing of replacing the blood pump, which ensures safety on the one hand and avoids waste of consumables on the other.

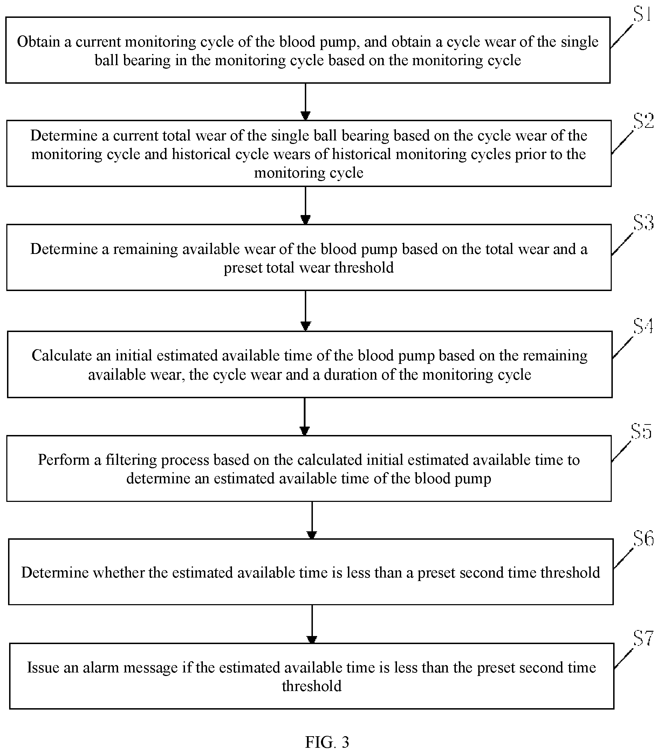

With reference to FIG. 3, in some embodiments, after step S5, the method further includes the following steps:

S6. Determine whether the estimated available time is less than a preset second time threshold.

In a specific implementation, it is determined whether the estimated available time is less than a preset second time threshold. The second time threshold can be set by a person skilled in the art, and the present invention does not specifically limit it. The purpose of setting the second time threshold is to provide sufficient preparation time for the staff. The second time threshold may be set in such a way of comprehensively considering the preparation time for preparing the blood pump, the preparation time for handling shutdown replacement, and the like.

S7. Issue an alarm message if the estimated available time is less than the preset second time threshold.

In a specific implementation, if the estimated available time is less than a preset second time threshold, it means that the blood pump is about to be damaged, so an alarm message is issued to prompt the user to replace the blood pump.

The following is a complete implementation of the above algorithm: when we get a brand new blood pump, the software design monitoring cycle is 10 minutes; the auxiliary prediction life function is turned on in the display panel. After we start the pump, a pop-up box pops up to ask if it is a new blood pump, if it is confirmed to be a new blood pump, the timing starts. In the 10th minute of the pump operation, we get the flow value, the pressure value before the pump is -71 mmHg, and the pressure value after the pump is 132 mmHg; thus $\triangle P$ = 203 mmHg$\approx$0.027 MPa, and the speed is 4500 r/min. Assume that the wear coefficients are k1=0.01x10^(-9) m^3/(N.m), $k_2$=0.008x10^(-9) m^3/(N.m), the other parameters are assumed to be R=0.005 m, D_e=0.02 m, d_h=0.05 m, and F_1=8 N, a=$\angle$30°, t=10 min, also assume the most severe wear, is a=a1, then substitute the above known parameters into the formula,

$$y = (k_1 \frac{n*(F_1 - \frac{\pi}{4}(D_e^2 - d_h^2)\triangle P)*sin2a}{40R(1-cos^3 a_1)} t \; + \; k_2 \frac{n(F_1 - \frac{\pi}{4}(D_e^2 - d_h^2)\triangle P)\sin 2a}{40R(1- COS^3 a_1)} t) \cos a$$

so as to calculate the wear height (cycle wear) in the 10-minute cycle to be approximately 2.94e-06 m. Suppose we set the wearable distance to 0.006 m, then calculate the wear degree to be 0.049%. The display accuracy on the display panel is percentile, and the display value is 0. The estimated usable time is calculated to be 339.96 hours, and then the estimated wear degree is displayed on the display panel. At this time, because the data calculated in the previous round is 0, the estimated usable time is not displayed after filtering, In the 20th minute of the pump operation, we obtain the flow value, the pressure value before the pump was -70 mmHg, the pressure value after the pump was 133 mmHg, and the other parameters do not change. The speed and the known parameters are substituted into the formula to calculate the wear height within the 10 minutes to be about 2.94e-06 m, adding the wear in the first 10 minutes, the current total wear is about 5.88e-06 m. The wear degree is then calculated and updated to the display panel as 0.098%, and the remaining available time is calculated to be 339.8 hours. Assuming that the preset time threshold is 10 times the cycle, that is, the time threshold is 100 minutes, the software determines that the difference between the estimated available time calculated this time and the value of the 10th minute is within 100 minutes (that is, the cycle is less than the time threshold), that is, the machine is determined to be in a stable operating state, and then the estimated available time parameters are calculated and the display panel display value is updated.

The above monitoring cycle of 10 minutes is only an example, which can be 1s, 1 min, etc., and can be set according to actual needs. The flow value, pressure value, and speed obtained can be the weighted arithmetic mean of each parameter within the monitoring cycle, or the harmonic mean, etc., which can more truly reflect the algorithm within the monitoring cycle.

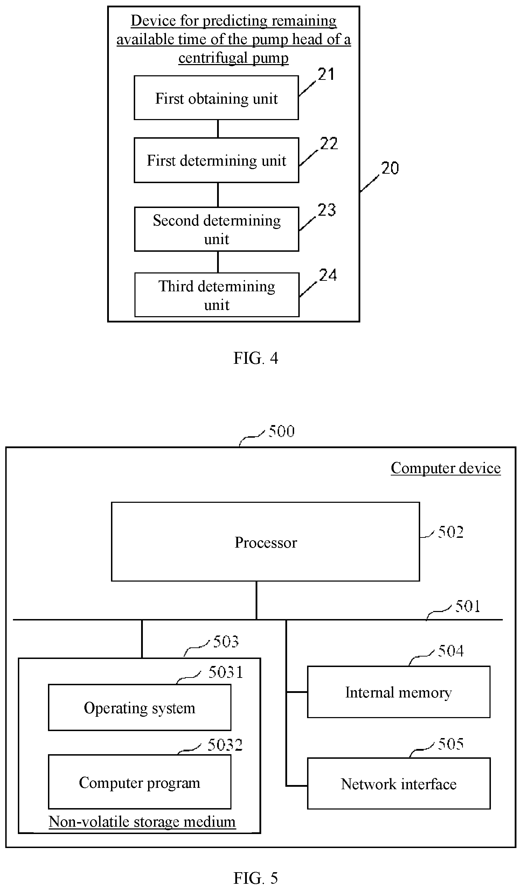

With reference to FIG. 4, FIG. 4 is a schematic block diagram of a device for predicting a blood pump remaining available time of a centrifugal pump provided in an embodiment of the present invention. Corresponding to the above method for predicting a blood pump remaining available time of a centrifugal pump, the present invention also provides a device 20 for predicting a blood pump remaining available time of a centrifugal pump. The device 20 for predicting a blood pump remaining available time of a centrifugal pump includes units for executing the above method for predicting a blood pump remaining available time of a centrifugal pump. This device 20 for predicting a blood pump remaining available time of a centrifugal pump can be configured in a desktop computer, tablet computer, laptop computer, a terminal, and the like. Specifically, device 20 for predicting a blood pump remaining available time of a centrifugal pump includes:

A first obtaining unit 21, which is configured to obtain a current monitoring cycle of the blood pump, and obtain a cycle wear of the single ball bearing in the monitoring cycle based on the monitoring cycle;

A first determining unit 22, which is configured to determine a current total wear of the single ball bearing based on the cycle wear of the monitoring cycle and historical cycle wears of historical monitoring cycles prior to the monitoring cycle;

A second determining unit 23, which is configured to determine a remaining available wear of the blood pump based on the total wear and a preset total wear threshold;

A calculating unit 24, which is configured to calculate an initial estimated available time of the blood pump based on the remaining available wear, the cycle wear, and a duration of the monitoring cycle; and

A third determining unit 25, which is configured to perform a filtering process based on the calculated initial estimated available time to determine an estimated available time of the blood pump.

In one embodiment, the performing of the filtering process based on the calculated initial estimated available time to determine the estimated available time of the blood pump comprises: obtaining a historical estimated available time corresponding to a preceding historical monitoring cycle of the monitoring cycle; determining whether a difference between the historical estimated available time and the initial estimated available time is less than a preset first time threshold; and using the initial estimated available time as the estimated available time of the blood pump if the difference between the historical estimated available time and the estimated available time is smaller than the preset first time threshold.

In one embodiment, the obtaining of the cycle wear of the single ball bearing in a preset monitoring cycle comprises: obtaining a target speed value of the blood pump and a target pressure difference between an output port and an input port of the blood pump during the monitoring cycle; and determining the cycle wear based on the target speed value, the target pressure difference and the duration of the monitoring cycle.

In one embodiment, the obtaining of the target speed value of the blood pump and the target pressure difference between the output port and the input port of the blood pump during the monitoring cycle comprises: obtaining a weighted arithmetic mean or harmonic mean of speed values of the blood pump during the monitoring cycle as the target speed value; and obtaining a weighted arithmetic mean or harmonic mean of pressure differences between the output port and the input port of the blood pump during the monitoring cycle as the target pressure difference.

In one embodiment, the determining of the cycle wear based on the target speed value, the target pressure difference and the duration of the monitoring cycle comprises: obtaining a preset mapping relationship between pre-stored speed values, pressure difference value as well as durations and wears; and determining the cycle wear corresponding to the target speed value, the target pressure difference value and the duration of the monitoring cycle based on the preset mapping relationship.

In one embodiment, the determining of the total wear of the single ball bearing based on the cycle wear of the monitoring cycle and the historical cycle wears of the historical monitoring cycles prior to the monitoring cycle comprises: obtaining the total wear by accumulating and summing the cycle wear and all the historical cycle wears.

In one embodiment, the determining of the initial estimated available time of the blood pump based on the remaining available wear, the cycle wear and the duration of the monitoring cycle comprises: calculating a ratio of the remaining available wear to the cycle wear; and calculating a product of the ratio and the duration of the monitoring cycle to obtain the initial estimated available time of the blood pump.

In one embodiment, the device 20 for predicting a blood pump remaining available time of a centrifugal pump further comprises: a second determining unit, which is configured to determine whether the estimated available time is less than a preset second time threshold; and an alarm unit, which is configured to issue an alarm message if the estimated available time is less than the preset second time threshold.

It should be noted that a person skilled in the art can clearly understand that the specific implementation process of the above-mentioned device 20 for predicting a blood pump remaining available time of a centrifugal pump and each unit thereof can refer to the corresponding description in the above-mentioned method embodiments. For the convenience and simplicity of description, they will not be repeated herein.

The above-mentioned device 20 for predicting a blood pump remaining available time of a centrifugal pump can be implemented in the form of a computer program, and the computer program can be run on a computer device as shown in FIG 5.

With reference to FIG. 5, FIG. 5 is a schematic block diagram of a computer device provided in an embodiment of the present invention.

The computer device 500 may be a terminal or a server, where the terminal may be an electronic device with communication function such as a smart phone, a tablet computer, a laptop computer, a desktop computer, a personal digital assistant, and a wearable device etc. The server may be an independent server or a server cluster composed of multiple servers.

The computer device 500 includes a processor 502, a memory, and a network interface 505 connected via a system bus 501, where the memory may include a non-volatile storage medium 503 and an internal memory 504.

The non-volatile storage medium 503 may store an operating system 5031 and a computer program 5032. When the computer program 5032 is executed, the processor 502 may execute a method for predicting the remaining available time of a centrifugal pump blood pump.

The processor 502 is configured to provide computing and control capabilities to support the operation of the entire computer device 500.

The internal memory 504 provides an environment for the operation of the computer program 5032 in the non-volatile storage medium 503. When the computer program 5032 is executed by the processor 502, the processor 502 can execute a method for predicting the blood pump remaining available time of a centrifugal pump.

The network interface 505 is used for network communication with other devices. a person skilled in the art can understand that the above structure is only a block diagram of a part of the structure related to the scheme of the present application, and does not constitute a limitation on the computer device 500 to which the scheme of the present application is applied. The specific computer device 500 may include more or less components than those shown in the figure, or combine certain components, or have different component arrangements.

In this case, the processor 502 is configured to run the computer program 5032 stored in the memory to implement the steps of the method for predicting a blood pump remaining available time of a centrifugal pump as proposed in any of the above method embodiments.

It should be understood that in the embodiments of the present application, the processor 502 may be a central processing unit (CPU), and the processor 502 may also be other types of general-purpose processors, digital signal processors (DSP), application-specific integrated circuits (ASIC), field-programmable gate arrays (FPGA) or other programmable logic devices, discrete gates or transistor logic devices, discrete hardware components, etc. Among them, the general-purpose processor may be a microprocessor or the processor may also be any conventional processor, etc.

It can be understood by a person of ordinary skill in the art that all or part of the processes in the method for implementing the above embodiment can be completed by instructing the relevant hardware through a computer program. The computer program may be stored in a storage medium, which is a computer-readable storage medium. The computer program is executed by at least one processor in the computer system to implement the process steps of the embodiments of the above method.

Therefore, the present invention also provides a storage medium. The storage medium may be a computer-readable storage medium. The storage medium stores a computer program. When the computer program is executed by a processor, the processor is caused to execute the steps of the method for predicting a blood pump remaining available time of a centrifugal pump as proposed in any of the above method embodiments.

The storage medium is a physical, non-transient storage medium, such as a USB flash drive, a mobile hard disk, a read-only memory (ROM), a magnetic disk or an optical disk, and other physical storage media that can store program codes. The computer-readable storage medium can be non-volatile or volatile.

A person of ordinary skill in the art can realize that the units and algorithm steps of each example described in the embodiments disclosed herein can be implemented by electronic hardware, computer software, or a combination of the two. In order to clearly illustrate the interchangeability of hardware and software, the composition and steps of each example have been generally described in terms of function in the above description. Whether these functions are executed in hardware or software depends on the specific application and design constraints of the technical solution. A person skilled in the art can use different methods to implement the described functions for each specific application, but such implementation should not be considered to be beyond the scope of the present invention.

In the several embodiments provided by the present invention, it should be understood that the disclosed device and method can be implemented in other ways. For example, the device embodiments described above are only illustrative. For example, the division of each unit is only a logical function division, and there may be other division methods in actual implementation. For example, multiple units or components can be combined or integrated into another system, or some features can be omitted or not executed.

The steps in the method of the embodiments of the present invention can be adjusted in order, combined and deleted according to actual needs. The units in the device of the embodiments of the present invention can be combined, divided and deleted according to actual needs. In addition, the functional units in the various embodiments of the present invention can be integrated into a processing unit, or each unit can exist physically separately, or two or more units can be integrated into one unit.

If the integrated unit is implemented in the form of a software functional unit and sold or used as an independent product, it can be stored in a storage medium. Based on this understanding, the technical solution of the present invention, or the part that contributes to the existing technology, or all or part of the technical solution, can be embodied in the form of a software product. The computer software product is stored in a storage medium and includes several instructions for enabling a computer device (which can be a personal computer, terminal, or network device, etc.) to execute all or part of the steps of the method described in each embodiment of the present invention.

In the above embodiments, the description of each embodiment has its own emphasis. For the part that is not described in detail in a certain embodiment, reference may be made to the relevant description of other embodiments.

Obviously, a person skilled in the art can make various changes and modifications to the present invention without departing from the principles and scope of the present invention. In this way, if these modifications and variations of the present invention fall within the scope of the claims of the present invention and its equivalent technology, the present invention is also intended to include these modifications and variations.

The above is only certain specific implementation of the present invention, but the protection scope of the present invention is not limited to this. A person skilled in the art can easily think of various equivalent modifications or replacements within the technical scope disclosed by the present invention. These modifications or replacements should be covered within the protection scope of the present invention. Therefore, the protection scope of the present invention shall be based on the protection scope of the claims.

**Claims**

1. A method for predicting a blood pump remaining available time of a centrifugal pump, **characterized in that** the blood pump comprises a rotor and a housing, and the rotor and the housing are connected by a single ball bearing, the method comprising:

   obtaining a duration of a current monitoring cycle of the blood pump;
   obtaining a current cycle wear of the single ball bearing in the current monitoring cycle based on the current monitoring cycle;
   determining a current total wear of the single ball bearing based on the current cycle wear of the current monitoring cycle and historical cycle wears of historical monitoring cycles prior to the current monitoring cycle;
   determining a remaining available wear of the blood pump based on the current total wear and a preset total wear threshold;
   calculating an initial estimated available time of the blood pump based on the remaining available wear, the current cycle wear, and the duration of the current monitoring cycle; and
   performing a filtering process based on the calculated initial estimated available time to determine an ultimate estimated available time of the blood pump.

2. The method for predicting the blood pump remaining available time of the centrifugal pump according to claim 1, **characterized in that** the performing of the filtering process based on the calculated initial estimated available time to determine the ultimate estimated available time of the blood pump comprises:

   obtaining a historical estimated available time corresponding to a preceding historical monitoring cycle of the current monitoring cycle;
   determining whether a difference between the historical estimated available time and the initial estimated available time is less than a preset first time threshold; and
   using the initial estimated available time as the ultimate estimated available time of the blood pump in response to the difference between the historical estimated available time and the estimated available time being smaller than the preset first time threshold.

3. The method for predicting the blood pump remaining available time of the centrifugal pump according to claim 1, **characterized in that** the obtaining of the current cycle wear of the single ball bearing in a preset monitoring cycle comprises:

   obtaining a target speed value of the blood pump and a target pressure difference between an output port and an input port of the blood pump during the current monitoring cycle; and
   determining the current cycle wear based on the target speed value, the target pressure difference, and the duration of the monitoring cycle.

4. The method for predicting the blood pump remaining available time of the centrifugal pump according to claim 3, **characterized in that** the obtaining of the target speed value of the blood pump and the target pressure difference between the output port and the input port of the blood pump during the monitoring cycle comprises:

   obtaining a weighted arithmetic mean or harmonic mean of speed values of the blood pump during the current monitoring cycle as the target speed value; and
   obtaining a weighted arithmetic mean or harmonic mean of pressure differences between the output port and the input port of the blood pump during the current monitoring cycle as the target pressure difference.

5. The method for predicting the blood pump remaining available time of the centrifugal pump according to claim 3, **characterized in that** the determining of the current cycle wear based on the target speed value, the target pressure difference and the duration of the monitoring cycle comprises:

> obtaining a preset mapping relationship between pre-stored speed values, pressure difference value as well as durations and wears; and
> determining the current cycle wear corresponding to the target speed value, the target pressure difference value and the duration of the monitoring cycle based on the preset mapping relationship.

6. The method for predicting the blood pump remaining available time of the centrifugal pump according to claim 1, **characterized in that** the determining of the total wear of the single ball bearing based on the current cycle wear of the current monitoring cycle and the historical cycle wears of the historical monitoring cycles prior to the current monitoring cycle comprises:
obtaining the total wear by accumulating and summing the current cycle wear and all the historical cycle wears.

7. The method for predicting the blood pump remaining available time of the centrifugal pump according to claim 1, **characterized in that** the determining of the initial estimated available time of the blood pump based on the remaining available wear, the current cycle wear, and the duration of the monitoring cycle comprises:

> calculating a ratio of the remaining available wear to the current cycle wear; and
> calculating a product of the ratio and the duration of the monitoring cycle to obtain the initial estimated available time of the blood pump.

8. The method for predicting the blood pump remaining available time of the centrifugal pump according to claim 1, **characterized in that** the method further comprises:

> determining whether the ultimate estimated available time is less than a preset second time threshold; and
> issuing an alarm message if the ultimate estimated available time is less than the preset second time threshold.

9. A device for predicting a blood pump remaining available time of the centrifugal pump, **characterized in that** the device comprises units for executing the method according to claim 1.

10. A computer device for predicting a blood pump remaining available time of a centrifugal pump, wherein the blood pump comprises a rotor and a housing, and the rotor and the housing are connected by a single ball bearing, the device comprising:

> a processor;
> a memory connected to the processor, the memory including a computer program that, when executed by the processor, causes the processor to:

>> obtain a duration of a current monitoring cycle of the blood pump;
>> obtain a current cycle wear of the single ball bearing in the current monitoring cycle based on the current monitoring cycle;
>> determine a current total wear of the single ball bearing based on the current cycle wear of the current monitoring cycle and historical cycle wears of historical monitoring cycles prior to the current monitoring cycle;
>> determine a remaining available wear of the blood pump based on the current total wear and a preset total wear threshold;
>> calculate an initial estimated available time of the blood pump based on the remaining available wear, the current cycle wear, and the duration of the monitoring cycle; and
>> perform a filtering process based on the calculated initial estimated available time to determine an ultimate estimated available time of the blood pump.

11. A computer-readable storage medium that stores a computer program for predicting a blood pump remaining available time of a centrifugal pump, wherein the blood pump comprises a rotor and a housing, and the rotor and the housing are connected by a single ball bearing, the computer program including instructions to:

> obtain a duration of a current monitoring cycle of the blood pump;
> obtain a current cycle wear of the single ball bearing in the current monitoring cycle based on the current monitoring cycle;

determine a current total wear of the single ball bearing based on the current cycle wear of the current monitoring cycle and historical cycle wears of historical monitoring cycles prior to the current monitoring cycle;

determine a remaining available wear of the blood pump based on the current total wear and a preset total wear threshold;

calculate an initial estimated available time of the blood pump based on the remaining available wear, the current cycle wear, and the duration of the monitoring cycle; and

perform a filtering process based on the calculated initial estimated available time to determine an ultimate estimated available time of the blood pump.

Obtain a current monitoring cycle of the blood pump, and obtain a cycle wear of the single ball bearing in the monitoring cycle based on the monitoring cycle
/S1

Determine a current total wear of the single ball bearing based on the cycle wear of the monitoring cycle and historical cycle wears of historical monitoring cycles prior to the monitoring cycle
/S2

Determine a remaining available wear of the blood pump based on the total wear and a preset total wear threshold
/S3

Calculate an initial estimated available time of the blood pump based on the remaining available wear, the cycle wear and a duration of the monitoring cycle
/S4

Perform a filtering process based on the calculated initial estimated available time to determine an estimated available time of the blood pump
/S5

FIG. 1

FIG. 2

Obtain a current monitoring cycle of the blood pump, and obtain a cycle wear of the single ball bearing in the monitoring cycle based on the monitoring cycle *S1*

Determine a current total wear of the single ball bearing based on the cycle wear of the monitoring cycle and historical cycle wears of historical monitoring cycles prior to the monitoring cycle *S2*

Determine a remaining available wear of the blood pump based on the total wear and a preset total wear threshold *S3*

Calculate an initial estimated available time of the blood pump based on the remaining available wear, the cycle wear and a duration of the monitoring cycle *S4*

Perform a filtering process based on the calculated initial estimated available time to determine an estimated available time of the blood pump *S5*

Determine whether the estimated available time is less than a preset second time threshold *S6*

Issue an alarm message if the estimated available time is less than the preset second time threshold *S7*

FIG. 3

Device for predicting remaining
available time of the pump head of a
centrifugal pump

First obtaining unit — 21

First determining unit — 22

Second determining
unit — 23

Third determining
unit — 24

20

FIG. 4

500

Computer device

Processor — 502

501

503

5031

Operating system

5032

Computer program

Non-volatile storage medium

504

Internal memory

505

Network interface

FIG. 5

**Europäisches Patentamt**
**European Patent Office**
**Office européen des brevets**

# EUROPEAN SEARCH REPORT

Application Number

EP 25 16 2698

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CN 106 089 753 B (UNIV TAIYUAN TECHNOLOGY) 19 January 2018 (2018-01-19) * the whole document * | 1-11 | INV. A61M1/36 A61M60/109 A61M60/232 |
| A,D | CN 110 259 702 A (TIANCHANG YUANAN MACHINERY CO LTD) 20 September 2019 (2019-09-20) * the whole document * | 1-11 | A61M60/38 A61M60/508 A61M60/585 A61M60/825 |
| A | US 2021/346678 A1 (BAUMBACH HARDY [DE] ET AL) 11 November 2021 (2021-11-11) * paragraph [0004] * * paragraph [0007] * * paragraph [0032] - paragraph [0038] * * paragraph [0041] * * paragraph [0050] - paragraph [0054] * * paragraph [0061] - paragraph [0067] * * figures 1-6 * | 1-11 | F04D15/00 F04D15/02 F04D29/046 G16H40/20 |

TECHNICAL FIELDS SEARCHED (IPC)

A61M
G16H
F04D

The present search report has been drawn up for all claims

| Place of search | Date of completion of the search | Examiner |
|---|---|---|
| The Hague | 29 July 2025 | Kempeneers, Johanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 25 16 2698

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2025

| Patent document cited in search report | | Publication date | Patent family member(s) | Publication date |
|---|---|---|---|---|
| CN 106089753 | B | 19-01-2018 | NONE | |
| CN 110259702 | A | 20-09-2019 | NONE | |
| US 2021346678 | A1 | 11-11-2021 | DE 102018210076 A1 | 24-12-2019 |
| | | | DE 112019003117 A5 | 11-03-2021 |
| | | | JP 7610833 B2 | 09-01-2025 |
| | | | JP 2021528167 A | 21-10-2021 |
| | | | JP 2025013950 A | 28-01-2025 |
| | | | US 2021346678 A1 | 11-11-2021 |
| | | | US 2025032773 A1 | 30-01-2025 |
| | | | WO 2019243582 A1 | 26-12-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 111249551 B
- CN 113446259 A
- CN 112915293 A
- CN 115300785 A
- WO 2021051645 A2
- CN 106089753 B
- CN 110259702 A

**Non-patent literature cited in the description**

- Analysis of Wear Characteristics of Spherical Friction Pairs. Mining Machinery Press